(19) Europäisches Patentamt
European Patent Office
Office européen des brevets

(11) **EP 2 912 439 B1**

(12) **EUROPÄISCHE PATENTSCHRIFT**

(45) Veröffentlichungstag und Bekanntmachung des Hinweises auf die Patenterteilung:
**01.08.2018 Patentblatt 2018/31**

(21) Anmeldenummer: **13789176.8**

(22) Anmeldetag: **24.10.2013**

(51) Int Cl.:
*B60K 15/04* (2006.01)     *B60K 15/03* (2006.01)
*F02D 41/14* (2006.01)     *F02D 19/06* (2006.01)
*F02D 41/00* (2006.01)     *G01N 33/28* (2006.01)
*G01N 21/64* (2006.01)

(86) Internationale Anmeldenummer:
**PCT/EP2013/003209**

(87) Internationale Veröffentlichungsnummer:
**WO 2014/063823 (01.05.2014 Gazette 2014/18)**

(54) **ANORDNUNG UND VERFAHREN FÜR EIN KRAFTFAHRZEUG ZUM ERFASSEN EINER KRAFTSTOFFSORTE UND/ODER KRAFTSTOFFCHARAKTERISTIK**

ARRAY AND METHOD FOR A MOTOR VEHICLE FOR DETECTING A FUEL TYPE AND/OR A FUEL CHARACTERISTIC

ENSEMBLE ET PROCÉDÉ DE DÉTERMINATION D'UN TYPE DE CARBURANT ET/OU D'UNE CARACTÉRISTIQUE DU CARBURANT POUR UN VÉHICULE À MOTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.10.2012 DE 102012020913**

(43) Veröffentlichungstag der Anmeldung:
**02.09.2015 Patentblatt 2015/36**

(73) Patentinhaber: **Hochschule für angewandte Wissenschaften Fachhochschule Coburg 96450 Coburg (DE)**

(72) Erfinder:
• **BÄR, Ferdinand 96242 Sonnefeld (DE)**
• **FAN, Zhu 96450 Coburg (DE)**
• **KRAHL, Jürgen 96450 Coburg (DE)**

(74) Vertreter: **FDST Patentanwälte Nordostpark 16 90411 Nürnberg (DE)**

(56) Entgegenhaltungen:
EP-B1- 1 629 265     WO-A1-2011/132079
US-A1- 2009 303 466     US-A1- 2009 317 299

**Beschreibung**

Die vorliegende Erfindung betrifft eine Anordnung für ein Kraftfahrzeug zum Erfassen einer Kraftstoffsorte und/oder einer Kraftstoffcharakteristik und ein Kraftfahrzeug umfassend die Anordnung. Des Weiteren betrifft die Erfindung ein Verfahren zur Bestimmung einer Zusammensetzung aus verschiedenen Kraftstoffen in einem Tank eines Kraftfahrzeugs.

Auf dem Markt sind verschiedenste Kraftstoffsorten von unterschiedlichen Herstellern erhältlich. Je nach Art des Kraftstoffes, beispielsweise Diesel oder Biodiesel, und je nach Hersteller weisen die Kraftstoffe unterschiedliche Charakteristika auf. Um die Kraftstoffe möglichst effizient und umweltverträglich zu nutzen, ist es wünschenswert, einen Verbrennungsmotor in Abhängigkeit des getankten Kraftstoffes zu steuern bzw. zu regeln. Die Dokumente US-A-2009/303466 und WO-A-2011/132079 zeigen Anordnungen zum Erfassen einer Kraftstoffcharakteristik. Das Dokument EP-B-1629265 zeigt eine Anordnung zur Detektion eines Betriebsstoffs einer Maschine. Es ist Aufgabe vorliegender Erfindung, eine Anordnung für ein Kraftfahrzeug anzugeben, die bei kostengünstiger Herstellung und wartungsarmem Betrieb ein möglichst genaues Erfassen einer getankten Kraftstoffsorte und/oder einer Kraftstoffcharakteristik des getankten Kraftstoffes ermöglicht. Des Weiteren ist es Aufgabe vorliegender Erfindung, ein Verfahren zur Bestimmung einer Zusammensetzung aus verschiedenen Kraftstoffen in einem Tank eines Kraftfahrzeugs anzugeben. Das Verfahren soll bei möglichst einfacher Durchführung die Kraftstoffzusammensetzung im Tank möglichst genau bestimmen.

Die Lösung der Aufgabe erfolgt durch die Merkmale der unabhängigen Ansprüche. Die abhängigen Ansprüche haben vorteilhafte Weiterbildungen der Erfindung zum Gegenstand.

Somit wird die Aufgabe gelöst durch eine Anordnung für ein Kraftfahrzeug zum Erfassen einer Kraftstoffsorte und/oder einer Kraftstoffcharakteristik. Die Anordnung umfasst eine Rohrleitung zur Anordnung zwischen einem Tankdeckel und einem Tank Kraftstoffsorte und/oder einer Kraftstoffcharakteristik des getankten Kraftstoffes ausgebildet. Erfindungsgemäß wird nicht die Kraftstoffsorte bzw. die Kraftstoffcharakteristik im Tank erfasst, sondern es erfolgt ein Erfassen der Kraftstoffsorte bzw. der Kraftstoffcharakteristik während der Kraftstoff durch die Rohrleitung in den Tank fließt. Dadurch ist es möglich, exakt den getankten Kraftstoff bzw. die Kraftstoffcharakteristik des getankten Kraftstoffes anstatt die Kraftstoffmischung bzw. die Kraftstoffcharakteristik einer Kraftstoffmischung im Tank zu erfassen. Die Erfassungseinheit ist zum Emittieren von Licht in den Kraftstoff ausgebildet. Insbesondere wird Laserlicht in den Kraftstoff emittiert. Des Weiteren ist die Erfassungseinheit zum Detektieren eines vom Kraftstoff ausgesendeten Fluoreszenzlichts ausgebildet. Erfindungsgemäß wird der Kraftstoff innerhalb der Rohrleitung zum Aussenden von Fluoreszenzlicht angeregt. Anhand des vom Kraftstoff ausgesendeten Fluoreszenzlichts kann die getankte Kraftstoffsorte und/oder eine Kraftstoffcharakteristik des getankten Kraftstoffes bestimmt werden.

Bevorzugt ist vorgesehen, dass die Erfassungseinheit eine Emittereinheit und eine Detektoreinheit aufweist. Die Emittereinheit ist vorzugsweise als erste Diode ausgebildet und dient zum Emittieren des Lichtes, insbesondere des Laserlichtes. Die Detektoreinheit ist vorzugsweise als zweite Diode ausgebildet und wird zum Detektieren des vom Kraftstoff ausgesendeten Fluoreszenzlichts genutzt. Alternativ zur Verwendung von zwei Dioden können auch lichtleitende Elemente genutzt werden, die das Licht in die Rohrleitung hineinleiten und das ausgesendete Fluoreszenzlicht aufnehmen. Des Weiteren ist es vorgesehen, dass die Erfassungseinheit lediglich einen in die Rohrleitung führenden Lichtleiter umfasst. Über diesen einen Lichtleiter kann sowohl das Licht in den Kraftstoff emittiert werden als auch das vom Kraftstoff ausgesendete Fluoreszenzlicht von der Rohrleitung nach außen abgeleitet werden.

Des Weiteren sind bevorzugt mehrere Erfassungseinheiten in der Rohrleitung integriert. Die mehreren Erfassungseinheiten werden vorzugsweise entlang des Umfangs der Rohrleitung verteilt. Besonders bevorzugt sind drei Erfassungseinheiten vorgesehen, die um jeweils 120° zueinander versetzt entlang des Umfangs der Rohrleitung angeordnet sind. Durch die versetzte Anordnung der Erfassungseinheiten ist eine gegenseitige Störung der Erfassungseinheiten ausgeschlossen und durch die Verwendung mehrerer Erfassungseinheiten ist eine Redundanz gegeben, die beispielsweise bei Ausfall oder Verschmutzung einer Erfassungseinheit von Vorteil ist.

Für die Integration der zumindest einen Erfassungseinheit in die Rohrleitung sind zwei bevorzugte Varianten vorgesehen: in einer ersten Variante ist in der Rohrleitung eine Aussparung, insbesondere ein Durchgangsloch, ausgebildet. In diese Aussparung wird die Erfassungseinheit eingesetzt. Insbesondere ist vorgesehen, dass in der Rohrleitung Durchgangslöcher zum Einsetzen der Dioden der Erfassungseinheit ausgebildet sind.

In einer zweiten Variante wird ein Rohrabschnitt der Rohrleitung aus einem lichtdurchlässigen Material gefertigt. Insbesondere wird in die Rohrleitung ein Rohrabschnitt aus Quarzglas eingesetzt. Außen an den Rohrabschnitt aus lichtdurchlässigem Material wird die zumindest eine Erfassungseinheit aufgesetzt. Die zweite Variante hat den Vorteil, dass der Kraftstoff in der Rohrleitung vollständig von der Erfassungseinheit getrennt ist, mit anderen Worten steht der Kraftstoff nicht in direktem Kontakt mit der Erfassungseinheit. Die Innenseite des Rohrabschnittes aus lichtdurchlässigem Material ist bevorzugt mit einer Antischmutzbeschichtung versehen.

Die erfindungsgemäße Anordnung umfasst bevorzugt eine Auswerteeinheit. Die Auswerteeinheit ist mit der zumindest einen Erfassungseinheit verbunden und dient zur spektroskopischen Auswertung des detektieren Fluoreszenzlichts. Die Auswerteeinheit ist ausgebildet, um laserinduzierte Fluoreszenzspektroskopie und/oder zeitaufgelöste laserinduzierten Fluoreszenzspektroskopie durchzuführen.

In der Auswerteeinheit werden bevorzugt Daten zu verschiedenen bekannten Kraftstoffsorten und/oder verschiedenen bekannten Kraftstoffcharakteristika gespeichert. Die Auswerteeinheit ist dazu ausgebildet, den erfassten Kraftstoff zumindest einer bekannten Kraftstoffsorte und/oder die erfasste Kraftstoffcharakteristik zumindest einer bekannten Kraftstoffcharakteristik zuzuordnen. Ein Vorteil der vorliegenden Erfindung ist, dass nicht ein Gemisch aus verschiedenen Kraftstoffen im Tank erfasst wird, sondern dass eine Erfassung des dem Tank zulaufenden Kraftstoffes in der Rohrleitung erfolgt. Dies hat den Vorteil, dass der zulaufende Kraftstoff eindeutig den am Markt befindlichen Kraftstoffen bzw. den in der Auswerteeinheit gespeicherten Kraftstoffen zuordbar ist.

Die Erfindung umfasst des Weiteren ein Kraftfahrzeug mit einer der oben beschriebenen Anordnungen. In dem Kraftfahrzeug mündet die Rohrleitung in einen Tank. Des Weiteren ist eine Mengenerfassungsvorrichtung vorgesehen. Die Mengenerfassungsvorrichtung dient zum Erfassen einer getankten Menge. Dies erfolgt durch Messen einer Durchlaufmenge des Kraftstoffes in der Rohrleitung und/oder durch Messen eines Füllstandes im Tank. Durch Erfassen der getankten Kraftstoffsorte und/oder einer Kraftstoffcharakteristik des getankten Kraftstoffes und durch Erfassen der zugetankten Kraftstoffmenge ist es möglich, stets die Kraftstoffzusammensetzung im Tank zu bestimmen. Dadurch kann ein Motorsteuergerät des Kraftfahrzeugs in Abhängigkeit von der Zusammensetzung des Kraftstoffes im Tank den Verbrennungsmotor steuern bzw. regeln.

Die Mengenerfassungsvorrichtung wird bevorzugt auch zum Einschalten der Erfassungseinheit genutzt. Sobald die Mengenerfassungsvorrichtung einen Betankungsvorgang erkennt wird die Erfassungseinheit aktiviert.

Bevorzugt ist es vorgesehen, mit der Erfassungseinheit auch eine Fehlbetankung, z.B. Diesel anstatt Benzin, zu erkennen und ein entsprechendes Signal, bevorzugt ein akustisches und/oder ein optisches Signal, auszugeben.

Die Erfindung umfasst des Weiteren ein Verfahren zur Bestimmung einer Kraftstoffzusammensetzung in einem Tank eines Kraftfahrzeugs. Die Kraftstoffzusammensetzung im Tank entsteht durch ein Vermischen verschiedener Kraftstoffe durch mehrere Tankvorgänge. Das Verfahren umfasst ein Erfassen einer Kraftstoffsorte und/oder einer Kraftstoffcharakteristik eines dem Tank zufließenden Kraftstoffes. Das Erfassen erfolgt dabei nicht im Tank sondern in einer Rohrleitung zwischen einem Tankdeckel und einem Tank. Des Weiteren erfolgt ein Erfassen einer getankten Kraftstoffmenge durch Messen einer Durchlaufmenge des Kraftstoffes in der Rohrleitung und/oder eines Füllstandes im Tank. In einem dritten Schritt erfolgt ein Berechnen der Kraftstoffzusammensetzung im Tank. Die Kraftstoffzusammensetzung gibt die erfasste Kraftstoffsorte und/oder Kraftstoffcharakteristik mit zugehöriger getankter Menge an.

Die im Rahmen der erfindungsgemäßen Anordnung und/oder des erfindungsgemäßen Kraftfahrzeugs beschriebenen vorteilhaften Ausgestaltungen und die Unteransprüche finden entsprechend vorteilhafte Anwendung auf das erfindungsgemäße Verfahren. Insbesondere ist bei dem erfindungsgemäßen Verfahren ebenfalls eine Erfassung einer Kraftstoffsorte und/der einer Kraftstoffcharakteristik über das vom Kraftstoff ausgesendete Fluoreszenzlicht vorgesehen.

Da sich unterschiedliche Kraftstoffe in ihrem Brenn-, Alterungs- und Emissionsverhalten stark voneinander unterscheiden können, ist es sinnvoll, die Motorsteuerung auf das aktuelle Kraftstoffgemisch im Tank anzupassen.

Biodiesel zeigt im Vergleich zu fossilem Dieselkraftstoff höhere Stickoxidemissionen und eine Anfälligkeit zur Ölschlammbildung durch Dieselkraftstoffeintrag in das Motoröl bei der Regeneration des Dieselpartikelfilters. Die Motorsteuerung kann bei bekannter Biodieselkonzentration dann so eingreifen, dass sich keine negativen Folgen aus einem hohen Biodieselgehalt ergeben.

Fossile Diesel- und Ottokraftstoffe können sich in ihrem Aromatengehalt und damit im Partikelemissionsverhalten stark voneinander unterscheiden. Durch eine gezielt geregelte, heißere oder kältere Verbrennung könnten diese Emissionen in Abhängigkeit der Zusammensetzung der Aromaten verringert werden. Dies ist z.B. durch die Steuerung des Einspritzverhaltens des Motors für Dieselkraftstoff gut steuerbar.

Der Ethanolgehalt in Ottokraftstoff hat einen enormen Einfluss auf das Kaltstartverhalten des Fahrzeugs. Außerdem kann bei bekannter Kraftstoffzusammensetzung von Ottokraftstoff der Klopfsensor des Motors entfallen. Auch dies kann vom Motorsteuergerät - bei bekannter Kraftstoffzusammensetzung im Tank - berücksichtigt werden.

Erfindungsgemäß müssen nicht die Fluoreszenzeigenschaften von unbekannten Kraftstoffgemischen bestimmt werden, sondern die Fluoreszenzeigenschaften von bekannten, am Markt erhältlichen bzw. in der Auswerteeinheit gespeicherten Kraftstoffen. Dabei kann sowohl das zeitliche Frequenzverhalten wie auch das spektrale Abklingverhalten erfasst werden, wodurch eine sehr präzise Kraftstofferkennung gewährleistet ist. Über die Mengenerfassungsvorrichtung des Fahrzeugs kann die Menge des getankten Kraftstoffes bestimmt und so die Zusammensetzung des Kraftstoffes im Tank errechnet werden, wodurch eine optimale Anpassung der Motorsteuerung oder -regelung entsprechend des Kraftstoffgemisches möglich wird.

Die spontane Emission von Lichtquanten aus einem elektronisch angeregten Zustand bezeichnet man allgemein als Fluoreszenz. Eine Fluoreszenzstrahlung tritt dann auf, wenn Elektronen aus dem Singulett-Grundzustand $S_0$ zunächst durch Absorption von Photonen in einen angeregten Zustand $S_i$ (i = 1, 2, 3, usw.) mit den Schwingungsniveaus 0, 1, 2, usw. übergehen und zunächst strahlungslos durch Schwingungsrelaxation (VR) von diesen einzelnen Niveaus auf das Schwingungsniveau 0 von $S_i$ zurückkehren. Durch innere Umwandlung findet dann ebenso strahlungslos der Übergang vom niedrigsten Schwingungsniveau eines höheren elektronischen Zustandes in ein Schwingungsniveau des nächst niederen Elektronenzustandes statt. Ist das Schwingungsniveau 0 von $S_1$ erreicht, gehen die Elektronen unter Aussendung von Fluoreszenzlicht in den Grundzustand $S_0$ über. Der Elektronenspin bleibt dabei erhalten (S=0). Die Energiedifferenz wird als Licht emittiert.

$$S_1 \rightarrow S_0 + hv$$

Die charakteristische Zeitkonstante, also die Zeitspanne von Anregung bis zur Beobachtung der Fluoreszenz, beträgt zwischen $\tau \approx 10^{-10}$ bis $10^{-6}$ s. Daraus folgt, dass die Lichterscheinung nach Abstellen der Anregungsquelle quasi sofort erlischt.

Voraussetzung für Fluoreszenz ist, dass das eingestrahlte Licht von Molekülen absorbiert werden kann und dadurch Elektronenübergänge induziert werden. Derartige Moleküle besitzen im Allgemeinen delokalisierte Elektronen in sogenannten bindenden $\pi$-Orbitalen. Diese sind in aromatischen Ringstrukturen zu finden. Schon bei dem einfachsten Vertreter der Verbindungsklasse der aromatischen Kohlenwasserstoffe, dem Benzol, ist der fluoreszenzspektroskopische Nachweis möglich.

Bei Ringstrukturen sind die $\pi$-Elektronen über das gesamte Molekül verteilt. Aufgrund der starren Struktur dieser Verbindungen sind diese weitgehend an Schwingungs- und Rotationsrelaxationen gehindert und neigen dazu, überschüssige Energie in Form von Licht wieder abzugeben.

Die Fluoreszenz eines Moleküls ist eine mehrdimensionale Größe, d.h. die Emission beinhaltet neben der wellenlängenaufgelösten Intensität noch weitere auswertbare Informationen, wie das Abklingverhalten und damit die Lebensdauer. Wie im sogenannten Jablonski-Diagramm ersichtlich ist, besitzen die einzelnen Deaktivierungsprozesse unterschiedliche Lebensdauern. Dieses Abklingverhalten der Emission kann durch eine einfache exponentielle Abkling-funktion mit der Lebensdauer $\tau$ beschrieben werden:

$$I_F = I_{F,t=0} \cdot \exp(- t / \tau)$$

Dieser Umstand wird in der zeitaufgelösten laserinduzierten Fluoreszenzspektroskopie (ZLIF) genutzt, sodass eine Unterscheidung von verschiedenen Kraftstoffen anhand ihres jeweiligen Abklingverhaltens möglich ist.

Bei nicht zeitaufgelösten laserinduzierten Fluoreszenzspektroskopie-Messungen (LIF-Messungen) erhält man die Messdaten in Form eines 2D-Spektrums. Dieses enthält die Signalintensität als Funktion der Wellenlänge. Durch kleine Zeitfenster und der Aufzeichnung einer Folge von 2D-Spektren lässt sich ein 3D-Spektrum erzeugen. Nach diesem Prinzip arbeiten LIF-Spektrometer mit ICCD Kamera als Detektor.

Die Erfassungseinheit und die Auswerteeinheit sind bevorzugt zur Durchführung dieser zeitaufgelösten laserinduzierten Fluoreszenzspektroskopie (ZLIF) und/oder laserinduzierten Fluoreszenzspektroskopie (LIF) ausgebildet.

Zum Unterscheiden kommerzieller Kraftstoffe unterschiedlicher Mineralölkonzerne werden die zeitaufgelösten Fluoreszenzeigenschaften der Kraftstoffe mittels ZLIF-Methode untersucht und in der Auswerteeinheit hinterlegt. Die Ergebnisse stellen die Fluoreszenzintensität als Funktion von den Emissionswellenlängen und den Abklingzeiten dar. Aus diesen Ergebnissen können viele Schlüssel-Variablen der Fluoreszenzeigenschaften von Kraftstoffen abgeleitet werden. Dies sind z.B. die maximale Intensität, die Wellenlänge für die maximale Fluoreszenzintensität oder das Frequenz- bzw. Abklingverhalten. Anhand dieser Variablen können Kraftstoffe klar unterschieden werden.

Es ist bekannt, dass die Fluoreszenz aus der Abstrahlung von Fluorophoren (z.B. aromatische Kohlenwasserstoffe AK oder polyzyklische aromatische Kohlenwasserstoffe PAK) herrührt und von nicht fluoreszierenden Substanzen (Alkane, Fettsäuremethylester (FAME), usw.) beeinflusst wird. Weiterhin wurde gefunden, dass die Kraftstoffe, die ähnliche zeitaufgelöste Fluoreszenzeigenschaften haben, vergleichbare Zusammensetzungen und Kraftstoffcharakteristika besitzen.

Die Messmatrix der ZLIF-Messungen besteht beispielsweise aus 55100 Messpunkten für jede Kraftstoffprobe. Die Matrix kann bevorzugt verkleinert werden, indem die Daten in Richtung der Zeitachse geeignet zerlegt werden, sodass keine wichtigen zeitlichen Informationen verloren gehen. Der einfachste Weg der zeitlichen Datenreduktion ist eine Integration der Fluoreszenz über den ganzen Zeitbereich für jede Wellenlänge. Dadurch wird ein klassisch nicht-zeitaufgelöstes 2D-Spektrum erlangt. Das heißt, dass das Fluoreszenzspektrum einer konventionellen laserinduzierten Fluoreszenzmessung (LIF) aus der ZLIF-Messung "extrahiert" werden kann.

Anstelle einer Integration über den ganzen Zeitbereich können zur Datenreduktion bevorzugt auch mehrere Integrationen über jeweils nur einen Teil des ganzen Zeitbereiches (z. B. jede Zeitzone mit 10 Nanosekunden) treten, bei dem die Fluoreszenz über die Zeitzone integriert wird. In der erfindungsgemäßen Anordnung werden z. B. die zeitaufgelösten Fluoreszenzspektren in 10 Zeitzonen unterteilt.

Die Datenreduktion vereinfacht die Zuordnung des getankten Kraftstoffes zu den in der Auswerteeinheit hinterlegten Daten.

Die ZLIF-Messung kann mit mehreren Zeitzonen die Fluoreszenzsignalgemische mittels der unterschiedlichen Lebensdauern von Fluorophoren besser unterscheiden. Dies ist besonders geeignet zum Unterscheiden komplexer Dieselkraftstoffe und Biodieselkraftstoffblends.

Bevorzugt werden vom zu detektierenden Kraftstoff wellenlängenselektiv zeitaufgelöste Fluoreszenzspektren erfasst, hieraus bei charakteristischen Wellenlängen jeweils die Lebensdauern bestimmt, und durch einen Vergleich der bestimmten Lebensdauern mit hinterlegten Lebensdauern auf eine bestimmte Kraftstoffsorte und/oder auf eine bestimmte Kraftstoffcharakteristik geschlossen. Es zeigt sich, dass ein solcher Vergleich eine eindeutige Zuordnung des Kraftstoffes und/oder der Kraftstoffcharakteristik ermöglicht.

In einer alternativen oder zusätzlichen vorteilhaften Variante werden vom zu detektierenden Kraftstoff wellenlängenselektiv zeitaufgelöste Fluoreszenzspektren erfasst und durch eine Hauptkomponentenanalyse der Messdaten auf eine bestimmte Kraftstoffsorte und/oder auf eine bestimmte Kraftstoffcharakteristik geschlossen. Eine Hauptkomponentenanalyse ermöglicht zur Bestimmung des Kraftstoffes und/oder der Kraftstoffcharakteristik eine Reduzierung der identifizierenden Variablen.

Es wurden ZLIF-Messungen von Biodieselkraftstoffblends aus dem CEC Referenzkraftstoff (DK9) und Biokraftstoff (frisches RME (Rapsöl-Methylester), gealtertes RME, RME mit Antioxidantien, PME (Palmöl-Methylester), KME (Kusumöl-Methlyester) und Hexan) zur Untersuchung und Unterscheidung der Kraftstoffblends durchgeführt.

Die Ergebnisse der LIF-Messungen von Biodieselkraftstoffblends können wie vorab beschrieben erlangt werden.

Zur Unterscheidung von Biodieselkraftstoffblends aus gleichen Kraftstoffen mit unterschiedlichem Verhältnis ist die LIF-Methode nicht geeignet. Durch ZLIF-Messungen ist dies jedoch ohne Probleme möglich.

Im Folgenden werden zwei Ausführungsbeispiele der Erfindung anhand der begleitenden Zeichnung im Detail erläutert. Dabei zeigen:

Fig. 1    eine Draufsicht und einen Schnitt zur erfindungsgemäßen Anordnung gemäß einem ersten Ausführungsbeispiel,

Fig. 2    eine Detailansicht zur Fig. 1,

Fig. 3    eine Draufsicht und einen Schnitt zur erfindungsgemäßen Anordnung gemäß einem zweiten Ausführungsbeispiel,

Fig. 4    eine Detailansicht zu Fig. 3,

Fig. 5    zeit- und wellenlängenaufgelöste (3D) Fluoreszenzspektren verschiedener Dieselkraftstoffe,

Fig. 6    einen Score-Plot aus einer Hauptkomponenten-Analyse der Fluoreszenzspektren gemäß Fig. 5,

Fig. 7    einen Stern-Volmer-Fit an die normierten Fluoreszenzintensitäten von unterschiedlichen Mischungen eines Dieselkraftstoffs mit einem Bioanteil, und

Fig. 8    einen linearen Fit an die auf einen reinen Bioanteil bezogenen Intensitäts-Messdaten entsprechend Fig. 7.

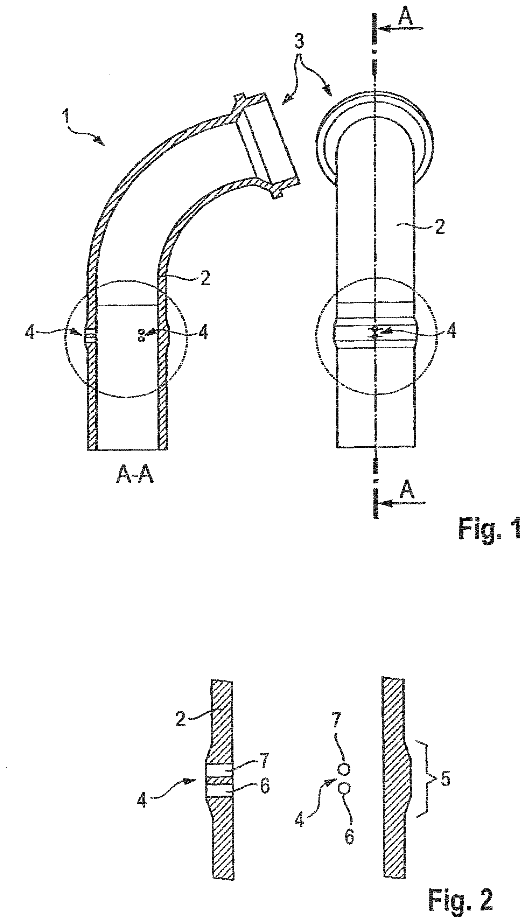

Im Folgenden wird anhand der Fig. 1 und 2 ein erstes Ausführungsbeispiel der erfindungsgemäßen Anordnung 1 für ein Kraftfahrzeug beschrieben.

Fig. 1 zeigt in Draufsicht und im Schnitt eine Rohrleitung 2 der Anordnung 1. Die Rohrleitung 2 ist im gezeigten Ausführungsbeispiel als ein Einfüllstutzen ausgebildet. Die Rohrleitung 2 weist einen Zulauf 3 auf. An dem Zulauf 3 wird ein Tankdeckel montiert. Über den Zulauf 3 wird Kraftstoff in die Rohrleitung 2 eingeführt. Entlang der Rohrleitung 2 läuft der Kraftstoff nach unten in Richtung eines Tanks eines Kraftfahrzeugs.

Fig. 2 zeigt das in Fig. 1 gekennzeichnete Detail. In der Rohrleitung 2 ist eine umlaufende Wulst 5 ausgebildet. In dieser Wulst 5 sind drei Erfassungseinheiten 4 integriert. Die Erfassungseinheiten 4 sind jeweils mit einem Winkel von 120° zueinander entlang des Umfangs der Rohrleitung angeordnet. Jede Erfassungseinheit 4 weist eine Emittereinheit 6 und eine Detektoreinheit 7 auf. Die Emittereinheit 6 ist als eine erste Diode ausgebildet. Die Detektoreinheit 7 ist als eine zweite Diode ausgebildet. Im ersten Ausführungsbeispiel sind pro Erfassungseinheit 4 zwei Durchgangslöcher in der Rohrleitung 2 vorgesehen. In den Durchgangslöchern werden die beiden Dioden für die Emittereinheit 6 und die Detektoreinheit 7 angeordnet.

Mittels der Emittereinheit 6 wird ein Laserlicht emittiert. Dieses Laserlicht regt den Kraftstoff in der Rohrleitung 2 zum fluoreszierenden Leuchten an. Das vom Kraftstoff ausgesendete Fluoreszenzlicht wird mit der Detektoreinheit 7 erfasst.

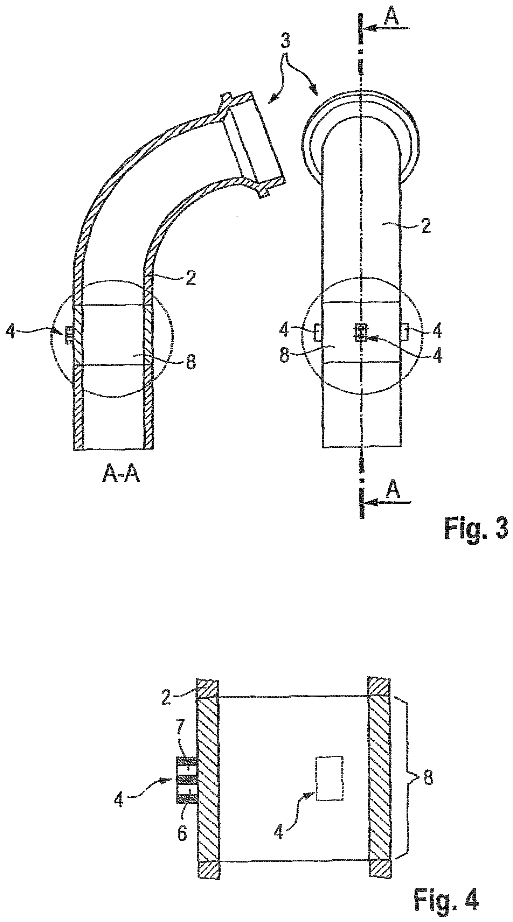

Die Fig. 3 und 4 zeigen das zweite Ausführungsbeispiel der Anordnung 1. Gleiche bzw. funktional gleiche Bauteile sind in allen Ausführungsbeispielen mit denselben Bezugszeichen versehen.

Im zweiten Ausführungsbeispiel ist in die Rohrleitung 2 ein Rohrabschnitt 8 eingesetzt. Der Rohrabschnitt 8 besteht aus einem lichtdurchlässigen Material, vorzugsweise aus Quarzglas. Der Rohrabschnitt 8 ist über seine gesamte Wandstärke lichtdurchlässig. Die Erfasssungseinheiten 4 sind außen auf den Rohrabschnitt 8 aufgesetzt. Die Innenfläche des Rohrabschnittes 8 ist mit einer schmutzabweisenden Schicht versehen. Im zweiten Ausführungsbeispiel emittiert die Emittereinheit 6 das Licht durch die Wand des Rohrabschnitts 8 hindurch in den Kraftstoff. Das ausgesendete Fluoreszenzlicht tritt durch die Wand des Rohrabschnitts 8 hindurch in die Detektoreinheit 7 ein.

Die Erfassungseinheiten 4 in beiden Ausführungsbeispielen werden mit einer Auswerteeinheit verbunden. In der Auswerteeinheit erfolgt eine Zuordnung des erfassten Kraftstoffes zu einer bekannten Kraftstoffsorte und/oder eine Zuordnung einer erfassten Kraftstoffcharakteristik zu einer bekannten Kraftstoffcharakteristik. Des Weiteren wird die Kraftstoff-Durchflussmenge in der Rohrleitung und/oder ein Füllstand in einem Tank gemessen. Dadurch ist es möglich, die genaue Kraftstoffzusammensetzung im Tank zu bestimmen und dementsprechend einen Motor des Kraftfahrzeugs anzusteuern oder zu regeln.

In der Anordnung 1 und in dem zugehörigen Verfahren wird die laserinduzierte, insbesondere die zeitaufgelöste laserinduzierte Fluoreszenz zum Erfassen eines Kraftstoffes und/oder einer Kraftstoffcharakteristik in der Rohrleitung 2, insbesondere im Einfüllstutzen, genutzt. Hierdurch lässt sich eine Vielzahl verschiedener Kraftstoffe zweifelsfrei voneinander unterscheiden. Biodieselanteile sowie der Alterungszustand des Kraftstoffes können ermittelt und zur Berechnung der Zusammensetzung des Kraftstoffes im Tank verwendet werden. Unbekannte Kraftstoffgemische, wie sie bei Kraftstoffmessungen direkt im Tank auftreten können, müssen mit dieser Methode nicht bestimmt werden. Zur Berechnung der Kraftstoffzusammensetzung werden die Daten des Tankfüllstandssensors oder der Kraftstoff-Durchlaufmenge in der Rohrleitung 2 herangezogen.

In Abhängigkeit von der Kraftstoffzusammensetzung im Tank des Kraftfahrzeugs kann ein im Motorsteuergerät hinterlegter Parametersatz aufgerufen werden, der Motorparameter wie Einspritzdruck, Einspritzzeit, Regenerationszyklus des Dieselpartikelfilters usw. der Zusammensetzung des Kraftstoffes anpasst.

Im Folgenden werden Ausführungsbeispiele der Erfindung anhand von Untersuchungsergebnissen näher erläutert.

Für einen einzigen Fluorophor kann die Lebensdauer bei der Emissionswellenlänge der maximalen Fluoreszenzintensität seine zeitaufgelösten Fluoreszenzeigenschaften am besten widerspiegeln. Für Fluorophorgemische, also insbesondere für Dieselkraftstoffgemische, können insbesondere die Lebensdauern aus den charakteristischen Emissionswellenlängen, welche die Peak-Wellenlängen aus dem Frequenzverhalten sind, ihre zeitaufgelösten Fluoreszenzeigenschaften zusammenfassen.

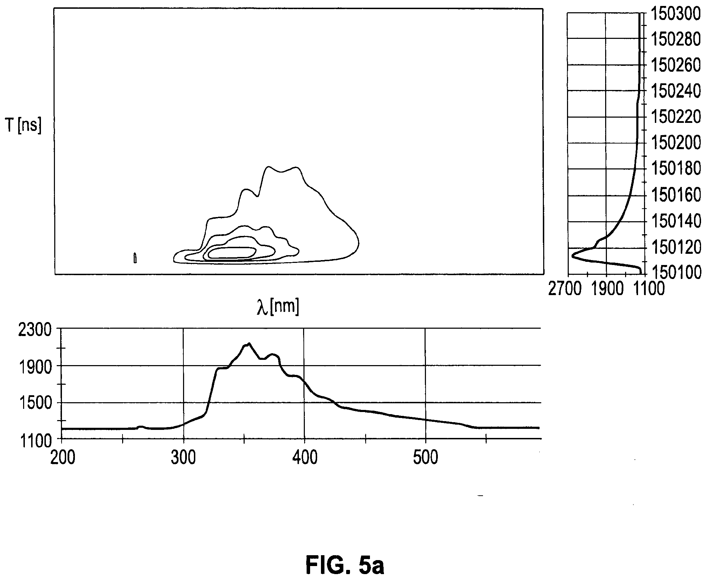

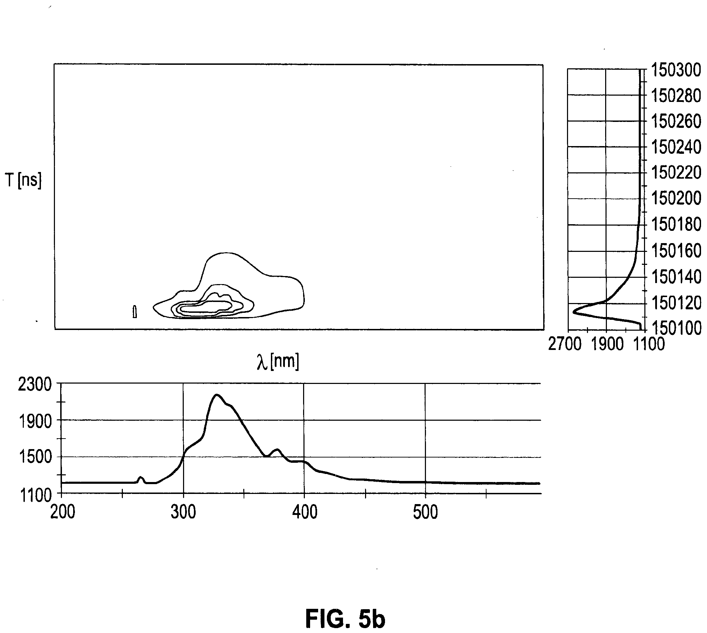

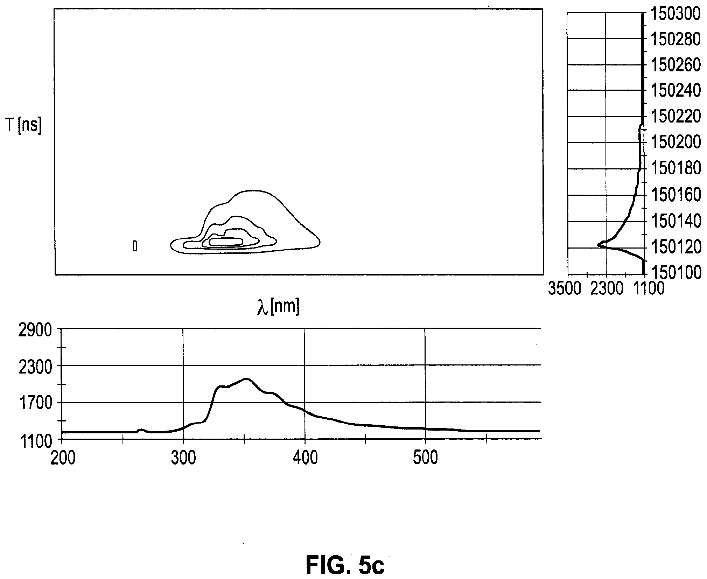

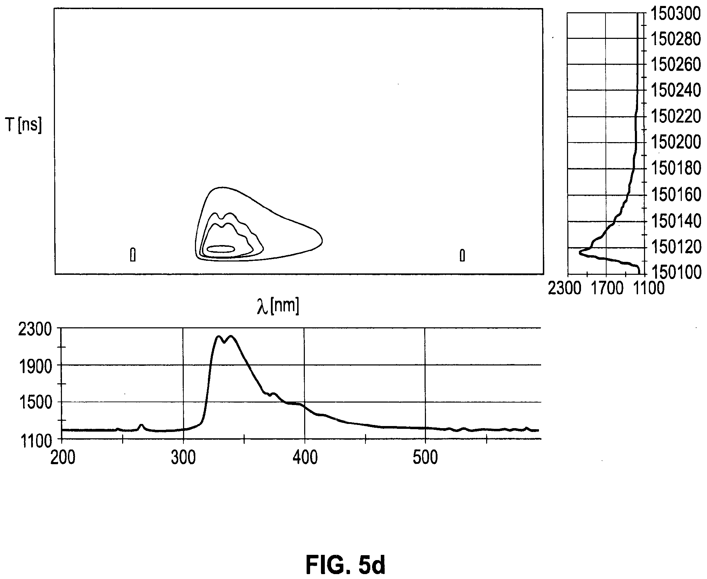

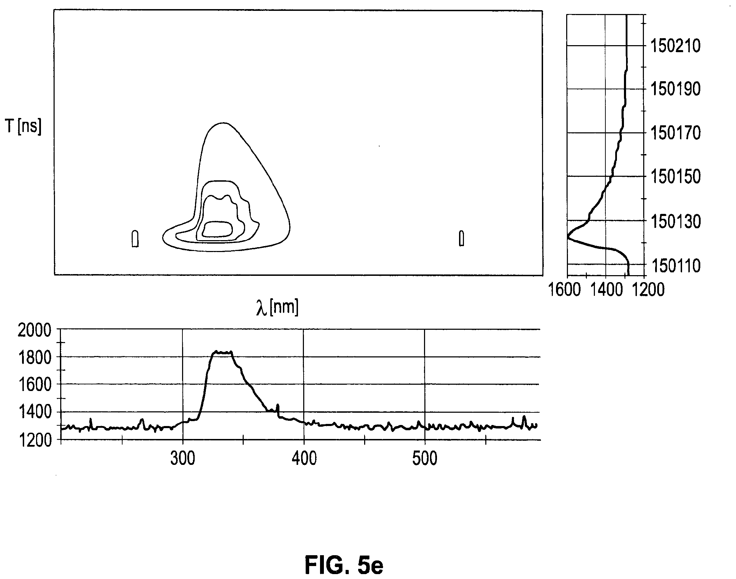

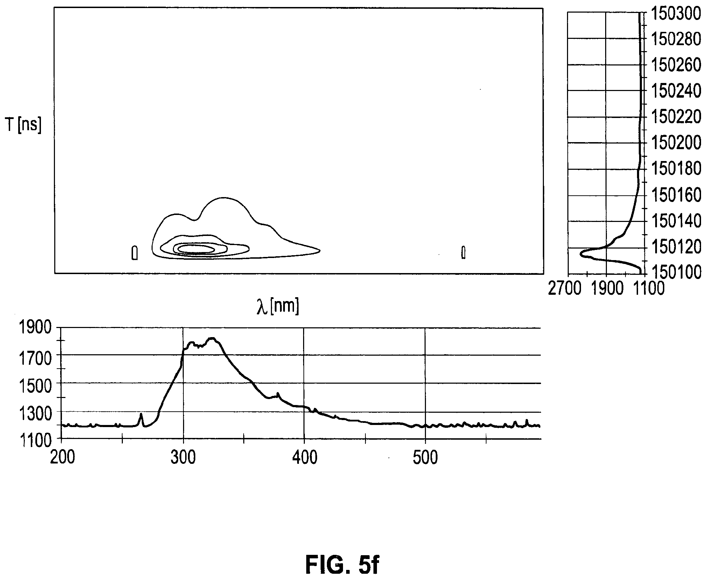

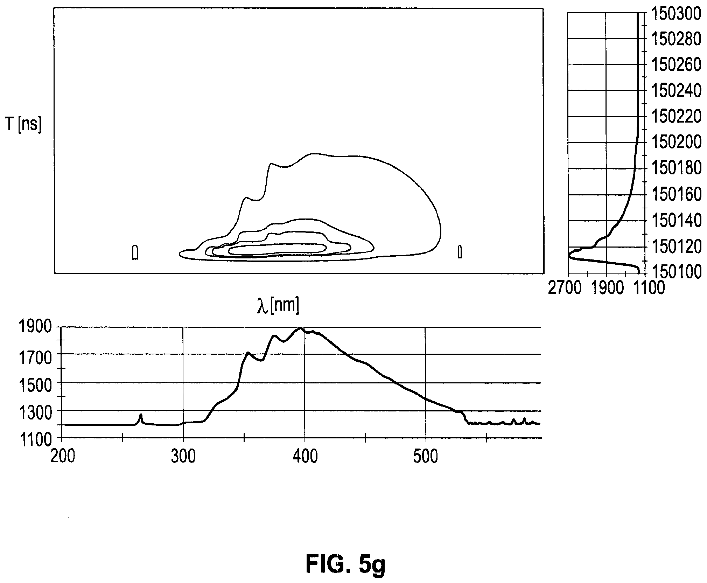

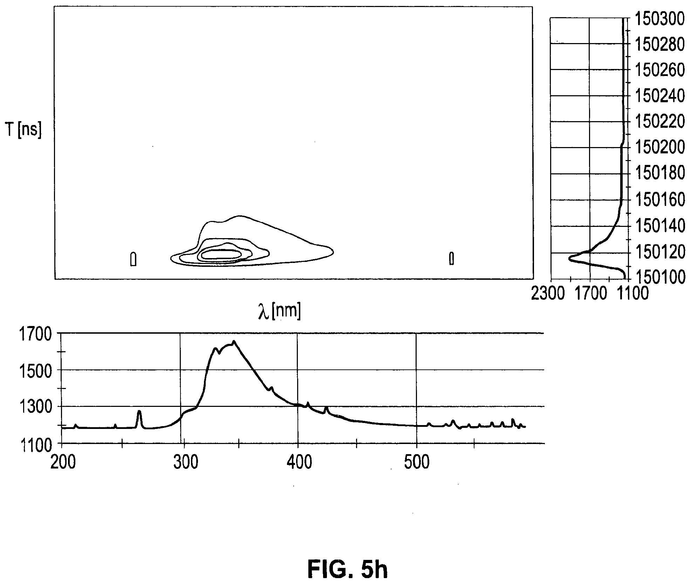

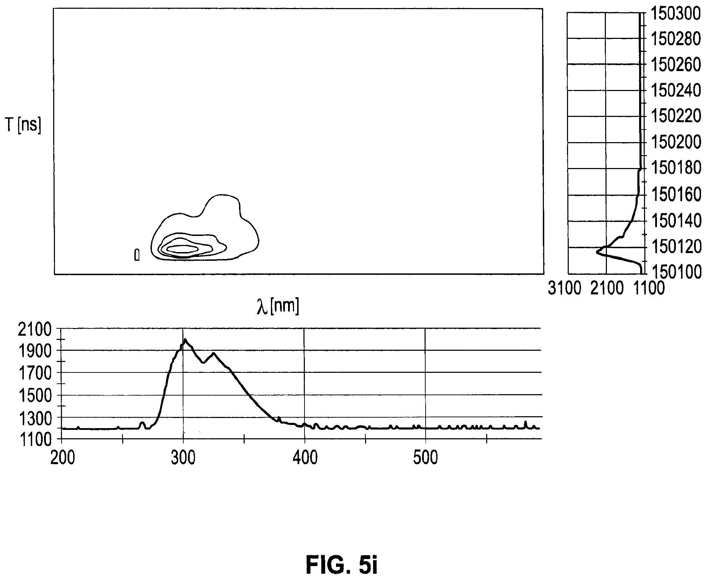

Zum Vergleich wurden neun Dieselkraftstoff/Biokraftstoffgemische mit der Methode der zeitaufgelösten laserinduzierten Fluoreszenzspektroskopie (ZLIF) gemessen und aus charakteristischen Emissionswellenlängen ihre Fluoreszenzlebensdauern bestimmt. Untersucht wurden marktübliche Dieselkraftstoffe von Tankstellen in Deutschland und im Ausland, neu entwickelte Dieselkraftstoffe wie Hydrierte Pflanzenöle (HVO), schwedischer Dieselkraftstoff MK1 und **einem** Forschungsblend des Thünen-Instituts für Agrartechnologie aus CEC Referenz Dieselkraftstoff, Biodiesel und Additiven. In den Figuren 5 sind die entsprechenden 3D-ZLIF-Spektren dargestellt, und zwar für Aral Diesel (Fig. 5a), Aral Ultimate Diesel (Fig. 5b), Shell V-Power Diesel (Fig. 5c), CEC Referenz-Dieselkraftstoff (Fig. 5d), Forschungsblend (Fig. 5e), HVO (Fig. 5f), CNPC Diesel Südchina (Fig. 5g), Diesel aus Argentinien (Fig. 5h) und schwedischen Dieselkraftstoff MK1 (Fig. 5i). Man erkennt jeweils die eigentlichen 3D-Plots der Intensitäten sowie die über die Zeit (Hochachse) bzw. über die Wellenlängen (Querachse) integrierten Spektren.

Die Wellenlängen, bei denen die Lebensdauern berechnet werden, werden anhand von Peak-Intensitäten aus diesen Diagrammen bestimmt. Damit verkörpern die ermittelten Lebensdauern die zeitaufgelösten Fluoreszenzeigenschaften der Kraftstoffe am besten. Durch eine Analyse der Fluoreszenzspektren der untersuchten Dieselkraftstoffe und Biodieselkraftstoffgemische konnten als neun charakteristische Emissionswellenlängen 300nm, 328nm, 335nm, 355nm, 377nm, 396nm, 407nm, 414nm und 423 nm ausgewählt werden. Die Lebensdauern bei diesen Wellenlängen wurden aus den jeweiligen Fluoreszenzabklingkurven berechnet. Es ergibt sich die nachfolgende Tabelle, die als Datenbank für die jeweiligen Dieselkraftstoffe hinterlegt wurde.

*Tabelle: Lebensdauern in ns von neun Dieselkraftstoffen und Biodieselkraftstoffgemischen bei charakteristischen Emissionswellenlängen*

| Dieselkraftstoffe | Charakteristische Emissionswellenlängen [nm] | | | | | | | | |
|---|---|---|---|---|---|---|---|---|---|
| | 300 | 328 | 335 | 355 | 377 | 396 | 407 | 414 | 423 |
| Aral Diesel | 14 | 12 | 12 | 19 | 29 | 37 | 36 | 39 | 41 |

(fortgesetzt)

| Dieselkraftstoffe | Charakteristische Emissionswellenlängen [nm] | | | | | | | | |
|---|---|---|---|---|---|---|---|---|---|
| | 300 | 328 | 335 | 355 | 377 | 396 | 407 | 414 | 423 |
| Aral Ultimate Diesel | 6 | 16 | 17 | 19 | 37 | 63 | 72 | 83 | 122 |
| Forschungsblend vom TI | 21 | 23 | 23 | 25 | 33 | 60 | 50 | 59 | 75 |
| Schwedischer Diesel | 11 | 22 | 26 | 31 | 32 | 39 | 35 | 39 | 53 |
| CEC Referenz-Diesel | 28 | 23 | 22 | 25 | 40 | 59 | 46 | 55 | 45 |
| HVO von Neste Oil | 12 | 17 | 19 | 21 | 23 | 23 | 23 | 23 | 24 |
| Diesel Südchina, CNPC | 32 | 8 | 8 | 15 | 21 | 23 | 23 | 23 | 24 |
| Argentinischer Diesel | 14 | 11 | 11 | 13 | 16 | 17 | 17 | 18 | 18 |
| Shell V-Power Diesel | 12 | 16 | 16 | 23 | 36 | 44 | 46 | 48 | 56 |

Es wird ersichtlich, dass aus einem Vergleich der bei Betankung eines Kraftfahrzeugs mittels ZLIF ermittelten Lebensdauern bei den charakteristischen Wellenlängen mit den in der Datenbank hinterlegten Werten auf den jeweiligen Dieselkraftstoff geschlossen werden kann.

Die Identifizierung der Kraftstoffe kann weiterhin ergänzend oder alternativ durch den Einsatz einer Hauptkomponentenanalyse (PCA) aus den ermittelten ZLIF-Messvariablen gemäß Fig. 5 erfolgen. Dabei werden die gewonnenen 3D-Messdaten der Kraftstoffe in eine Reihe von Werten von unkorrelierten Variablen (Hauptkomponenten PC genannt) orthogonal konvertiert. Mit anderen Worten werden die Koordinaten der originalen Lebensdauern für spezifische Wellenlängen (siehe oben) in ein neues Koordinatensystem der Hauptkomponenten konvertiert.

Nach einer Hauptkomponentenanalyse werden im neuen Koordinatensystem die Achsen anstelle der Lebensdauer mit den Hauptkomponenten bezeichnet und die Beiträge der Hauptkomponenten werden als die Score-Werte S definiert. Die Beziehung der Score-Werte S (Score-Matrix $\underline{S}$) und der originalen Variablenwerte X (Lebensdauermatrix $\underline{X}$) kann durch eine bekannte einfache Gleichung zur Orthogonalisierung beschrieben werden:

$$\underline{S} = \underline{X} \cdot \underline{L} \qquad\qquad (1)$$

Die Faktorladungsmatrix $\underline{L}$ ist die Matrix der Eigenvektoren (zueinander orthogonal) der Kovarianz von der originalen Variablen-Matrix $\underline{X}$, durch die das neue Koordinatensystem aufgebaut werden kann. Mit den entsprechenden Eigenwerten ($\lambda$) können außerdem die prozentualen Anteile (p) der Varianzen an der Gesamtvarianz sowie die kumulativen Anteile (P) berechnet werden:

$$p_k = \frac{\lambda_k}{\sum_{i=1}^{M} \lambda_i} \quad \text{und} \quad P_k = \frac{\sum_{i=1}^{k} \lambda_i}{\sum_{i=1}^{M} \lambda_i} = p_1 + p_2 + ... + p_k \qquad\qquad (2)$$

Zur Bestimmung der Zahl signifikanter Faktoren (z. B. die Zahl der Hauptkomponenten in der PCA) gibt es zahlreiche statistische Kriterien. Typische Methoden für die PCA sind beispielsweise: kumulativer Anteil der nötigen Varianzen an der Gesamtvarianz: es werden sukzessive Faktoren aufgenommen, bis ein bestimmter Anteil der Gesamtvarianz erreicht wurde (z. B. 95 %).

Die auf diese Weise entstandene erste Hauptkomponente hat die höchste Varianz. Die folgenden Hauptkomponenten (zweite, dritte, usw.) werden der Reihe nach der Varianz jeweils orthogonal (unkorreliert) zu der vorangegangenen Komponente herausgebildet. Die Grundannahme für die Verwendung der PCA zur Klassifikation und Dimensionsreduktion lautet hierbei, dass die Richtungen mit der größten Streuung (Varianz) die meiste Information beinhalten.

Deshalb können durch die ersten zwei oder drei Hauptkomponenten (PC1, PC2 und PC3) die wichtigsten Informationen aus den ZLIF-Spektren der Kraftstoffe herausgelesen werden. Dadurch wird die Unterscheidung der Kraftstoffe im Unterschied zu einem Vergleich der Lebensdauern bei einer Anzahl von charakteristischen Emissionswellenlängen vereinfacht.

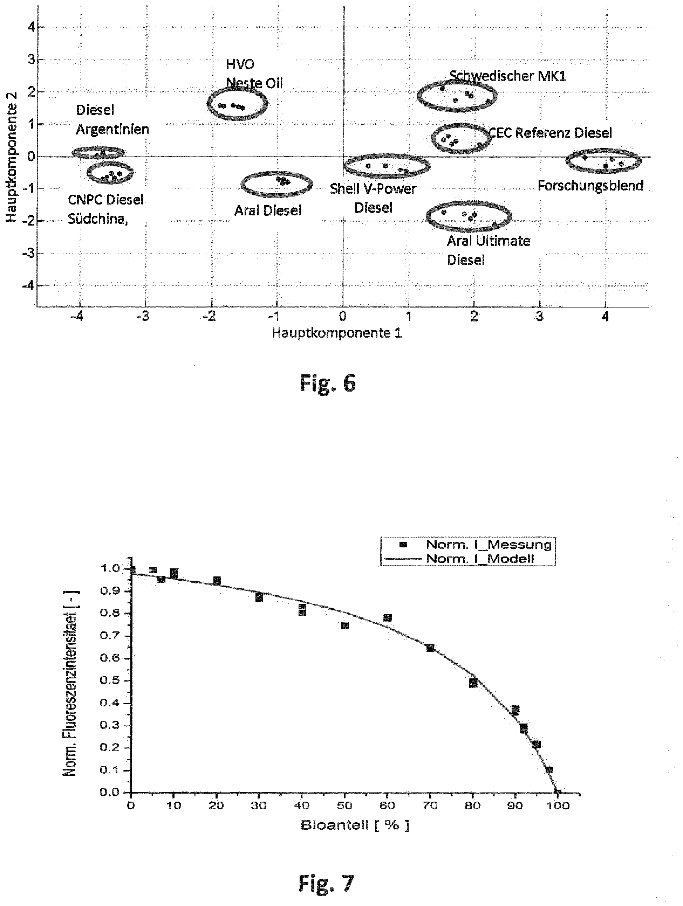

In Figur 6 ist beispielhaft der Score-Plot für die durch eine Hauptkomponentenanalyse der ZLIF-Messdaten gemäß Figur 5 ermittelten ersten zwei Hauptkomponenten (PC1 und PC2) gezeigt. Zur Reduzierung der statistischen Messfehler wurde jeder Dieselkraftstoff fünfmal gemessen. In Figur 6 sind somit für die verschiedenen Dieselkraftstoffe jeweils die "Anteile" (Score) der ersten beiden Hauptkomponenten eingetragen. Es sind zwei wichtige Informationen ersichtlich. Die Streuung der Score-Punkte innerhalb jedes Clusters ist sehr klein verglichen mit dem "Abstand" der Cluster zueinander. Des Weiteren sind die Score-Cluster aufgrund ihrer Lage gut zu unterscheiden. Das heißt, dass es möglich ist, die verschiedenen Dieselkraftstoffe und Biodieselkraftstoffgemische durch ihre Lebensdauer zu unterscheiden und zu klassifizieren. Im Score-Plot ist außerdem gut zu erkennen, dass die Score-Cluster für "Diesel Argentinien" und für "CNPC Diesel Südchina" nahe zusammen liegen und somit ähnliche Fluoreszenzeigenschaften besitzen. Dagegen gibt es die größten Unterschiede von ZLIF-Eigenschaften zwischen den beiden Dieselkraftstoffen und dem "Forschungsblend".

Durch Sammlung von ZLIF-Messdaten mehrerer typischer Dieselkraftstoffe und Biodieselkraftstoffgemische kann die Charakterisierung und Identifizierung der Dieselkraftstoffe oder Biodieselkraftstoffblends in einem großen Teil von Deutschland, der EU oder der ganzen Welt, erreicht werden.

Die Fluoreszenzeigenschaften (Frequenz- und Abklingverhalten) in Kraftstoffen und Kraftstoffgemischen sind von den Fluorophoren abhängig. Biodiesel und fossile Dieselkraftstoffe besitzen außerdem unterschiedliche Fluoreszenzintensitäten. Bei einer Anregungswellenlänge von 266 nm mit der Methode nach ZLIF kann Fluoreszenz für die Biokraftstoffe gemessen werden. Die Fluoreszenzintensität ist hauptsächlich vom fossilen Kraftstoffanteil abhängig, wie dies aus Figur 7 ersichtlich ist.

Die Fluoreszenzemission wiederum ist charakteristisch für die Konzentration der angeregten Fluorophore. Die Grundvoraussetzung für einen Einsatz zur quantifizierenden Messung der Kraftstoffverteilung, nämlich die Linearität des Signals zum Kraftstoffverhältnis, konnte in Untersuchungen, wie nachfolgend dargestellt, bestätigt werden. Damit wird es möglich, bereits bewährte Messroutinen zur Kalibrierung der Messungen anzuwenden.

Zunächst wurden die Kalibriermessungen des Fluoreszenz-Spektrometers mit einem Standard-Kraftstoff bei verschiedenen Konzentrationen durchgeführt. Danach wurde das Frequenz-und Abklingverhalten der verschiedenen Kraftstoffe, z. B. **von** CEC-Dieselkraftstoff mit einem Bioanteil von 0, 5, 7, 10, 20, 30, 40, 50, 60, 70, 80, 90, 91, 92, 95, 98, 100. gemessen. Die Abhängigkeit der Fluoreszenzintensität vom Bioanteil ist in Figur 7 dargestellt. Die Steigung nimmt mit höherem Bioanteil (niedrigerer fossiler Dieselkraftstoffanteil) zu, was auf eine typische Selbstfluoreszenzlöschung von Fluorophoren hindeutet.

Die Selbstfluoreszenzlöschung tritt häufig bei den Fluoreszenz-Messungen von hoher Fluorophor-Konzentration auf und kann durch Kollisionen von angeregten Molekülen im Grundzustand erfolgen. Das Ausmaß dieses Effektes ist abhängig von der Kollisionsfrequenz und daher umso größer je höher die Konzentration der Analyten ist. Dies kann zu einer negativen Steigung bei hoher Konzentration der Fluorophore führen. Das Fluoreszenzlöschungsverhalten kann durch die Stern-Volmer-Gleichung wie folgt beschrieben werden.

$$\frac{I_0}{I} = 1 + K_{SV} \cdot [Q] \qquad (3)$$

Mit: 10: Fluoreszenzintensität ohne Quencher, I: Fluoreszenzintensität mit Quencher, $K_{SV}$: Stern-Volmer-Konstante und [Q]: Quencher-Konzentration.

Für das Selbstfluoreszenzlöschungs-Modell ist die Fluorophor-Konzentration [Fl] gleich der Konzentration [Q] des Quenchers. Durch die Anpassung des Stern-Volmer-Modells mit den experimentellen Daten kann die Konstanten $K_{SV}$ bestimmt werden. Im Bereich des hohen Bioanteils, also in einem Bereich mit niedrigem Fluorophor-Anteil ist $I_0$ proportional zur Fluorophor-Konzentration. Figur 7 zeigt, dass die experimentellen Daten gut mit dem Stern-Volmer-Modell angepasst werden konnten. Die entsprechenden Konstanten können damit bestimmt werden mit $K_{SV}$ = 3,66 (stern-Volmer-Modell) und k = 4,56 (Steigung im Bereich hohen Bioanteils zwischen 90 und 100%).

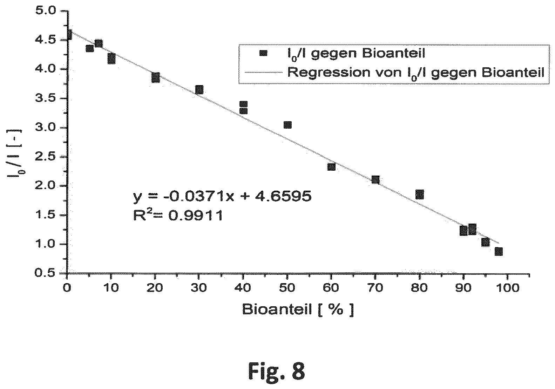

Durch Gleichung 3 wird aber auch gezeigt, dass es eine lineare Abhängigkeit zwischen $I_0/I$ und dem Bioanteil gibt. Der Bioanteil kann durch (1-[Fl]) umformuliert werden. Durch eine Prüfung der Linearität der Abhängigkeit zwischen $I_0/I$ und dem Bioanteil wurde bewertet, ob das Stern-Volmer-Modell bei der quantitativen Analyse der Biokraftstoffgemische aus Referenz CEC Dieselkraftstoff und RME angewendet werden kann. Das Ergebnis ist in Figur 8 dargestellt. Die gute lineare Beziehung ist in Figur 8 zu sehen. Es ist somit ersichtlich, dass das Stern-Volmer-Modell den Fluoreszenzlöschungseffekt durch den Anteil an fossilem Kraftstoffanteil gut beschreiben kann. Es ist somit möglich, den Bioanteil in Biodieselkraftstoffgemischen quantitativ zu bestimmen.

Bezugszeichenliste

1    Anordnung
2    Rohrleitung
3    Zulauf
4    Erfassungseinheit
5    Wulst
6    Emittereinheit
7    Detektoreinheit
8    Rohrabschnitt

**Patentansprüche**

1. Anordnung (1) für ein Kraftfahrzeug zum Erfassen einer Kraftstoffsorte und/oder einer Kraftstoffcharakteristik, umfassend

   - eine Rohrleitung (2) zur Anordnung zwischen einem Tankdeckel und einem Tank eines Kraftfahrzeugs, und
   - zumindest eine in die Rohrleitung (2) integrierte Erfassungseinheit (4) zum Erfassen der Kraftstoffsorte und/oder einer Kraftstoffcharakteristik eines durch die Rohrleitung laufenden Kraftstoffes,
   - wobei die Erfassungseinheit (4) zum Emittieren von Licht, vorzugsweise Laserlicht, in den Kraftstoff und zum Detektieren eines vom Kraftstoff ausgesendeten Fluoreszenzlichts ausgebildet ist,

   **dadurch gekennzeichnet, dass** eine Auswerteeinheit zur spektroskopischen Auswertung des detektierten Fluoreszenzlichts umfasst ist, die zur Durchführung von zeitaufgelöster, laserinduzierter, Fluoreszenzspektroskopie ausgebildet ist, und dass die Erfassungseinheit ausgebildet ist, wellenlängenselektiv zeitaufgelöste Fluoreszenzspektren zu erfassen, hieraus bei charakteristischen Wellenlängen jeweils die Lebensdauern zu bestimmen, und durch einen Vergleich der bestimmten Lebensdauern mit hinterlegten Lebensdauern auf eine bestimmte Kraftstoffsorte und/oder auf eine bestimmte Kraftstoffcharakteristik zu schließen.

2. Anordnung nach Anspruch 1, **dadurch gekennzeichnet, dass** die Erfassungseinheit (4) eine Emittereinheit (6), insbesondere eine erste Diode, zum Emittieren des Lichts und eine Detektoreinheit, insbesondere eine zweite Diode, zum Detektieren des Fluoreszenzlichts umfasst.

3. Anordnung nach einem der vorhergehenden Ansprüche, **dadurch gekennzeichnet, dass** mehrere Erfassungseinheiten (4) in die Rohrleitung (2) integriert sind.

4. Anordnung nach Anspruch 3, **dadurch gekennzeichnet, dass** die mehreren Erfassungseinheiten (4) entlang des Umfangs der Rohrleitung (2) verteilt sind.

5. Anordnung nach einem der vorhergehenden Ansprüche, **dadurch gekennzeichnet, dass** die zumindest eine Erfassungseinheit (4) in zumindest einer Aussparung, vorzugsweise in zumindest einem Durchgangsloch, in der Rohrleitung (2) angeordnet ist.

6. Anordnung nach einem der Ansprüche 1 bis 4, **dadurch gekennzeichnet, dass** ein Rohrabschnitt (8) der Rohrleitung (2) aus einem lichtdurchlässigen Material, vorzugsweise aus Quarzglas, besteht, wobei die zumindest eine Erfassungseinheit (4) außen auf den Rohrabschnitt (8) aufgesetzt ist.

7. Anordnung nach einem der vorhergehenden Ansprüche, **dadurch gekennzeichnet, dass** die Erfassungseinheit ausgebildet ist, wellenlängenselektiv zeitaufgelöste Fluoreszenzspektren zu erfassen und durch eine Hauptkomponentenanalyse der Messdaten auf eine bestimmte Kraftstoffsorte und/oder auf eine bestimmte Kraftstoffcharakteristik zu schließen.

8. Anordnung nach einem der vorhergehenden Ansprüche, **dadurch gekennzeichnet, dass** in der Auswerteeinheit Daten zu verschiedenen bekannten Kraftstoffsorten und/oder verschiedenen bekannten Kraftstoffcharakteristika gespeichert sind, wobei die Auswerteeinheit dazu ausgebildet ist, den erfassten Kraftstoff zumindest einer bekannten Kraftstoffsorte und/oder eine erfasste Kraftstoffcharakteristik zumindest einer bekannten Kraftstoffcharakteristik zuzuordnen.

9. Kraftfahrzeug, umfassend

- eine Anordnung (1) nach einem der vorhergehenden Ansprüche,
- einen Tank, in den die Rohrleitung (2) mündet,
- eine Mengenerfassungsvorrichtung zum Erfassen einer getankten Menge durch Messen einer Durchlaufmenge des Kraftstoffes in der Rohrleitung und/oder durch Messen eines Füllstandes im Tank, und
- ein Motorsteuergerät zum Steuern und/oder Regeln eines Motors in Abhängigkeit von der Zusammensetzung des Kraftstoffes im Tank.

10. Verfahren zur Bestimmung einer Zusammensetzung aus verschiedenen Kraftstoffen in einem Tank eines Kraftfahrzeugs, umfassend die folgenden Schritte:

- Erfassen einer Kraftstoffsorte und/oder einer Kraftstoffcharakteristik eines einem Tank zufließenden Kraftstoffes in einer Rohrleitung (2) zwischen einem Tankdeckel und einem Tank,
- Erfassen einer getankten Menge durch Messen einer Durchlaufmenge des Kraftstoffes in der Rohrleitung (2) und/oder eines Füllstandes im Tank, und
- Berechnen der Kraftstoffzusammensetzung im Tank in Anhängigkeit von der Kraftstoffsorte und/oder einer Kraftstoffcharakteristik mit zugehöriger getankter Menge,

**dadurch gekennzeichnet, dass** wellenlängenselektiv zeitaufgelöste Fluoreszenzspektren des zufließenden Kraftstoffes erfasst werden, und hieraus bei charakteristischen Wellenlängen jeweils die Lebensdauern bestimmt, sowie durch einen Vergleich der bestimmten Lebensdauern mit hinterlegten Lebensdauern auf eine bestimmte Kraftstoffsorte und/oder auf eine bestimmte Kraftstoffcharakteristik geschlossen wird.

11. Verfahren nach Anspruch 10, **dadurch gekennzeichnet, dass** die wellenlängenselektiv zeitaufgelöste Fluoreszenzspektren des zufließenden Kraftstoffes erfasst und durch eine Hauptkomponentenanalyse der Messdaten auf eine bestimmte Kraftstoffsorte und/oder auf eine bestimmte Kraftstoffcharakteristik geschlossen wird.

## Claims

1. Arrangement (1) for a motor vehicle for detecting a fuel type and/or a fuel characteristic, comprising

- a pipeline (2) to be arranged between a fuel tank cap and a tank of a motor vehicle, and
- at least one detection element (4) integrated into the pipeline (2) for detecting the fuel type and/or a fuel characteristic of a fuel running through the pipeline,
- the detection unit (4) being designed to emit light, preferably laser light, into the fuel and to detect a fluorescent light emitted by the fuel,

**characterized in that** an evaluation unit for the spectroscopic evaluation of the detected fluorescent light, which is configured to carry out time-resolved, laser-induced fluorescence spectroscopy, is comprised, and **in that** the detection unit is designed to detect wavelength-selectively time-resolved fluorescence spectra, from these in each case to determine the lifetimes at characteristic wavelengths and, by means of a comparison of the determined lifetimes with stored lifetimes, to draw conclusions about a specific fuel type and/or a specific fuel characteristic.

2. Arrangement according to Claim 1, **characterized in that** the detection unit (4) comprises an emitter unit (6), in particular a first diode, for emitting the light, and a detector unit, in particular a second diode, for detecting the fluorescent light.

3. Arrangement according to one of the preceding claims, **characterized in that** multiple detection units (4) are integrated in the pipeline (2).

4. Arrangement according to Claim 3, **characterized in that** the multiple detection units (4) are distributed along the circumference of the pipeline (2).

5. Arrangement according to one of the preceding claims, **characterized in that** the at least one detection unit (4) is arranged in at least one cut-out, preferably in at least one through-hole, in the pipeline (2).

6. Arrangement according to one of Claims 1 to 4, **characterized in that** a pipe section (8) of the pipeline (2) consists of a transparent material, preferably of quartz glass, wherein the at least one detection unit (4) is placed on the outside on the pipe section (8).

7. Arrangement according to one of the preceding claims, **characterized in that** the detection unit is designed to detect wavelength-selectively time-resolved fluorescence spectra and, by means of a main component analysis of the measured data, to draw conclusions about a specific fuel type and/or a specific fuel characteristic.

8. Arrangement according to one of the preceding claims, **characterized in that** data relating to various known fuel types and/or various known fuel characteristics are stored in the evaluation unit, wherein the evaluation unit is designed to assign the detected fuel to at least one known fuel type and/or a detected fuel characteristic to at least one known fuel characteristic.

9. Motor vehicle, comprising

- an arrangement (1) according to one of the preceding claims,
- a tank, into which the pipeline (2) opens,
- a volume detection device for detecting a volume put into the tank by measuring a flow rate of the fuel in the pipeline and/or by measuring a filling level in the tank, and
- an engine control device for the open-loop and/or closed-loop control of an engine on the basis of the composition of the fuel in the tank.

10. Method for determining a composition of various fuels in a tank of a motor vehicle, comprising the following steps:

- detecting a fuel type and/or a fuel characteristic of a fuel flowing to a tank in a pipeline (2) between a fuel tank cap and a tank,
- detecting a volume put into the tank by measuring a flow rate of the fuel in the pipeline (2) and/or a filling level in the tank, and
- calculating the fuel composition in the tank on the basis of the fuel type and/or a fuel characteristic with associated volume put into the tank,

**characterized in that** wavelength-selectively time-resolved fluorescence spectra of the fuel flowing in are detected and from these in each case the lifetimes are determined at characteristic wavelengths and, by means of a comparison of the determined lifetimes with stored lifetimes, conclusions are drawn about a specific fuel type and/or a specific fuel characteristic.

11. Method according to Claim 10, **characterized in that** the wavelength-selectively time-resolved fluorescence spectra of the fuel flowing in are detected and, by means of a main component analysis of the measured data, conclusions are drawn about a specific fuel type and/or about a specific fuel characteristic.

**Revendications**

1. Ensemble (1) destiné à un véhicule automobile afin de détecter un type de carburant et/ou une caractéristique du carburant, comprenant

- une tubulure (2) destinée à être disposée entre un bouchon de réservoir et un réservoir d'un véhicule automobile, et
- au moins une unité de détection (4) intégrée dans la tubulure (2) afin de détecter le type de carburant et/ou une caractéristique de carburant d'un carburant qui s'écoule dans la tubulure,
- dans lequel l'unité de détection (4) est adaptée pour émettre de la lumière, de préférence de la lumière laser, dans le carburant et pour détecter une lumière fluorescente émise par le carburant,

**caractérisé en ce qu'**il est prévu une unité d'évaluation pour l'évaluation spectroscopique de la lumière fluorescente détectée, qui est adaptée pour effectuer une spectroscopie de fluorescence induite par laser résolue dans le temps, et **en ce que** l'unité de détection est conçue pour détecter des spectres de fluorescence résolus dans le temps et sélectifs en longueur d'onde, afin de déterminer respectivement à partir de ceux-ci les durées de vie utile pour des longueurs d'onde caractéristiques et d'en déduire un type de carburant spécifique et/ou une caractéristique de carburant spécifique par une comparaison des durées de vie utile déterminées avec des durées de vie utile stockées.

2. Ensemble selon la revendication 1, **caractérisé en ce que** l'unité de détection (4) comprend une unité émettrice (6), en particulier une première diode, destinée à émettre la lumière et une unité à détecteur, en particulier une seconde diode, destinée à détecter la lumière fluorescente.

3. Ensemble selon l'une des revendications précédentes, **caractérisé en ce qu'**une pluralité d'unités de détection (4) sont intégrées dans la tubulure (2).

4. Ensemble selon la revendication 3, **caractérisé en ce que** la pluralité d'unités de détection (4) sont réparties le long de la circonférence de la tubulure (2).

5. Ensemble selon l'une des revendications précédentes, **caractérisé en ce que** ladite au moins une unité de détection (4) est disposée dans la tubulure (2), dans au moins un évidement, de préférence dans au moins un trou traversant.

6. Ensemble selon l'une des revendications 1 à 4, **caractérisé en ce qu'**un tronçon de tubulure (8) de la tubulure (2) est constitué d'un matériau transmettant la lumière, de préférence du verre de quartz, dans lequel ladite au moins une unité de détection (4) est placée à l'extérieur du tronçon de tubulure (8).

7. Ensemble selon l'une des revendications précédentes, **caractérisé en ce que** l'unité d'acquisition est conçue pour acquérir des spectres de fluorescence résolus dans le temps et sélectifs en longueur d'onde et pour en déduire un type de carburant spécifique et/ou une caractéristique de carburant spécifique par une analyse en composantes principales des données de mesure.

8. Ensemble selon l'une des revendications précédentes, **caractérisé en ce que** des données concernant différents types de carburant connus et/ou différentes caractéristiques de carburant connues sont stockées dans l'unité d'évaluation, dans lequel l'unité d'évaluation est adaptée pour associer le carburant détecté à au moins un type de carburant connu et/ou une caractéristique de carburant détectée à au moins une caractéristique de carburant connue.

9. Véhicule automobile, comprenant

   - un dispositif (1) selon l'une des revendications précédentes,
   - un réservoir dans lequel débouche la tubulure (2),
   - un dispositif de détection de quantité destiné à détecter une quantité de carburant en mesurant un débit de carburant dans la tubulure et/ou en mesurant un niveau de remplissage du réservoir, et
   - un appareil de commande de moteur destiné à commander et/ou à réguler un moteur en fonction de la composition du carburant dans le réservoir.

10. Procédé de détermination d'une composition de différents carburants dans le réservoir d'un véhicule automobile, comprenant les étapes suivantes :

    - détecter un type de carburant et/ou une caractéristique de carburant d'un carburant s'écoulant vers un réservoir dans une tubulure (2) entre un bouchon de réservoir et un réservoir,
    - détecter une quantité de carburant dans le réservoir en mesurant un débit de carburant dans la tubulure (2) et/ou un niveau de remplissage du réservoir, et
    - calculer la composition du carburant dans le réservoir en fonction du type de carburant et/ou d'une caractéristique du carburant avec une quantité associée dans le réservoir,

    **caractérisé en ce que** des spectres de fluorescence résolus dans le temps et sélectifs en longueur d'onde du carburant entrant sont détectés, **en ce que** les durées de vie utile sont respectivement déterminées à partir de ceux-ci pour des longueurs d'onde caractéristiques, et **en ce qu'**un type de carburant spécifique et/ou une caractéristique de carburant spécifique est déduite par une comparaison des durées de vie utile déterminées avec des durées de vie utile stockées.

11. Procédé selon la revendication 10, **caractérisé en ce que** les spectres de fluorescence résolus dans le temps et sélectifs en longueur d'onde du carburant entrant sont détectés et **en ce qu'**un type de carburant spécifique et/ou une caractéristique de carburant spécifique est déduite par une analyse en composantes principales des données de mesure.

A-A

Fig. 1

Fig. 2

A-A

Fig. 3

Fig. 4

T [ns]

λ [nm]

150300
150280
150260
150240
150220
150200
150180
150160
150140
150120
150100

2700  1900  1100

2300

1900

1500

1100
200        300        400        500

**FIG. 5a**

FIG. 5b

FIG. 5c

**FIG. 5d**

FIG. 5e

FIG. 5f

FIG. 5g

**FIG. 5h**

**FIG. 5i**

**Fig. 6**

**Fig. 7**

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009303466 A
- WO 2011132079 A
- EP 1629265 B